# EUROPEAN PATENT APPLICATION

(11) **EP 1 486 163 A1**
(43) Date of publication of application: **15.12.2004**
(21) Application number: 04012648.4
(22) Date of filing: 27.05.2004
(51) Int. Cl.: A61B 5/0404

(54) **Electroardiograph and electrocardiograph control method**

(30) Priority: 12.06.2003 JP 2003167833
(71) Applicant: Omron Healthcare Co., Ltd., Kyoto 615-0084 (JP)
(72) Inventor: Tanabe, Kazuhisa, c/o Omron Healthcare Co., Ltd., Ukyo-ku Kyoto-shi Kyoto 615-0084 (JP); Ishida, Junichi, c/o Omron Healthcare Co., Ltd., Ukyo-ku Kyoto-shi Kyoto 615-0084 (JP); Umeda, Masahiro, c/o Omron Healthcare Co., Ltd., Ukyo-ku Kyoto-shi Kyoto 615-0084 (JP); Yamamoto, Norihito, c/o Omron Healthcare Co., Ltd., Ukyo-ku Kyoto-shi Kyoto 615-0084 (JP); Moroki, Yoko, c/o Omron Healthcare Co., Ltd., Ukyo-ku Kyoto-shi Kyoto 615-0084 (JP)
(74) Representative: Kilian, Helmut, Dr.

(57) **Abstract**

An electrocardiograph and an electrocardiograph control method capable of recording the electrocardiographic waveform under satisfactory measurement conditions with a simple operation are disclosed. In the case where a measurement switch is depressed (MEASUREMENT SW in S10) in sleep mode, a CPU (10) starts the main power supply and sets the electrocardiograph in active mode and performs the initial operation for starting to control the various parts (S20). Further, the electrocardiograph selects a measurement program for executing the measurement function thereof, and the process before step S60 is skipped to branch to the measurement process.

## Description

### BACKGROUND OF THE INVENTION

### 1. Field of the Invention

The present invention relates to an electrocardiograph and an electrocardiograph control method, and in particular to an electrocardiograph and an electrocardiograph control method capable of recording a satisfactorily measured electrocardiographic waveform by a simple operation.

### 2. Description of the Related Art

An electrocardiogram is used as an effective method of diagnosing heart diseases such as myocardial infarct, angina cordis and the ischemic heart disease. In order to diagnose these heart diseases more effectively, the use of the electrocardiogram at the time of occurrence of an abnormality such as a chest pain or palpitation in daily life is desirable for early identification and proper treatment.

It may happen, however, that a hospital suitable for measuring the electrocardiographic waveform cannot be visited immediately after occurrence of an abnormality or that the abnormality has already ceased before the measurement begins in the hospital. In recent years, therefore, a portable electrocardiograph has been proposed to measure the electrocardiographic waveform accurately at the time of occurrence of an abnormality thereby making it possible to measure the electrocardiographic waveform whenever an abnormality occurs.

As a portable device like this, Japanese Unexamined Patent Publication No. 7-194562 (hereinafter referred to as Reference 1) discloses an electrocardiographic waveform measuring apparatus comprising an electrocardiographic wave measuring unit and a processing unit, wherein an electrode supplied with an electrocardiographic wave of the electrocardiographic wave measuring unit is used as a switch for starting the measurement, and the electrocardiographic wave data measured by the electrocardiographic wave measurement unit is output to the processing unit and processed in the processing unit thereby to simplify the measurement operation.

On the other hand, Japanese Unexamined Patent Publication No. 8-224216 (hereinafter referred to as Reference 2) discloses a portable biological signal measuring apparatus capable of preventing a malfunction using a configuration in which the depression of a switch for at least a predetermined time length is detected to switch between on state and off state.

In the case where the electrocardiographic waveform is measured using the electrocardiographic wave measuring apparatus disclosed in Reference 1, however, power is switched on by depressing a power button after which a measurement button is required to be depressed with one electrode pressed against the human body while holding the electrocardiographic electrode. This requires many steps of operation before starting the measurement after power on, often leading to the problem that the electrocardiographic waveform at the time of occurrence of an abnormality may fail to be recorded.

In the case where the electrocardiographic waveform is measured using the portable biological signal measuring apparatus disclosed in Reference 2, the measurement process is not started until switches such as a power switch and a measurement on/off switch required to input a measurement start instruction are kept depressed for at least a predetermined time length, often posing the problem that the electrocardiographic waveform at the time of occurrence of an abnormality may fail to be recorded.

### SUMMARY OF THE INVENTION

This invention has been achieved in view of the problems described above, and an object of the invention is to provide an electrocardiograph and an electrocardiograph control method in which the steps of operation before a predetermined mode is reached after switching on power of the apparatus are reduced and the electrocardiographic waveform can be measured in a satisfactory manner and recorded by a simple operation.

In order to achieve the object described above, according to one aspect of the invention, there is provided an electrocardiograph comprising a functional switch for designating a function, a storage unit for storing the program to implement the function, a power control means for controlling the power supply, and a control means wherein upon detection of the functional switch in power saving mode at least capable of detecting the depression of the functional switch, the control means controls the power control means to supply power to achieve the power state capable of implementing the function and performs control operation to execute the program stored in the storage unit for implementing the function corresponding to the functional switch of which the depression has been detected.

This function preferably includes the function of measuring the electrocardiographic wave in the electrocardiograph.

Further, preferably, the electrocardiograph comprises at least a pair of induction electrodes brought into contact with the body of the subject for measuring the electrocardiographic waveform of the subject, wherein the measurement switch making up the functional switch for designating the function of measuring the electrocardiographic waveform is arranged at a position adapted to be depressed when the induction electrode is brought into contact with the body of the subject. Once the induction electrodes are brought into contact with the body of the subject when power is off, therefore, the control means controls the power control means to supply power and performs control operation to execute the program stored in the storage unit to implement the measurement function.

According to another aspect of the invention, there is provided an electrocardiograph control method for controlling an electrocardiograph including a functional switch for designating a function and a storage unit for storing the program to implement the function, comprising the power control step for controlling the power supply, the first control step for controlling the power control step to be executed to supply power until the power mode capable of implementing the function is attained, upon detection of depression of the functional switch in the power saving mode where at least the depression of the functional switch can be detected, and the second control step for performing the control operation to execute the program stored in the storage unit for implementing a function corresponding to the function switch of which the depression has been detected.

### BRIEF DESCRIPTION OF THE DRAWINGS

Fig. 1 shows a front view of an electrocardiograph 1.
Fig. 2 shows a top plan view of the electrocardiograph 1.
Fig. 3 shows a left side view of the electrocardiograph 1.
Fig. 4 shows a right side view of the electrocardiograph 1.
Fig. 5 shows an example of the measurement position.
Fig. 6 shows a block diagram of the functional configuration of the electrocardiograph 1.
Fig. 7 shows a specific example of the functional configuration including a CPU 101, an operating unit 104, a memory 106, a power control unit 107 and a power supply unit 108.
Fig. 8 shows a flowchart for the menu process.
Fig. 9 shows a flowchart for the setting process.
Fig. 10 shows a flowchart for the display process.
Fig. 11 shows a flowchart for the measurement process.
Fig. 12 shows a flowchart of the overall process of the electrocardiograph 1.
Fig. 13 shows a front view of a specific example of the electrocardiograph 1 according to a modification.

### DESCRIPTION OF THE PREFERRED EMBODIMENTS

Embodiments of the invention are described below with reference to the accompanying drawings. In the following description, the same.parts and component elements are designated by the same reference numerals and have the same names and the same functions, respectively. Therefore, these parts and component elements are not explained repeatedly in detail.

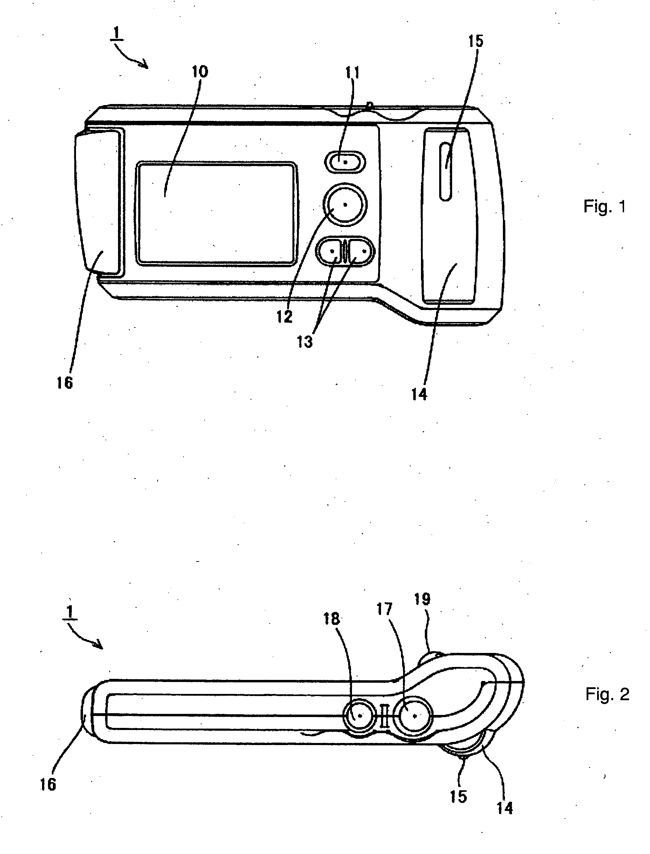

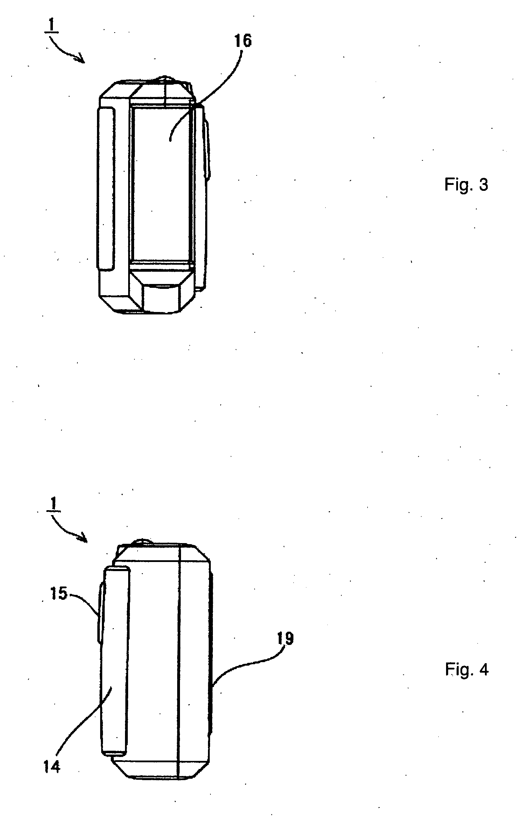

A specific example of the appearance of a portable electrocardiograph (hereinafter referred to as the electrocardiograph) 1 according to an embodiment is shown in Figs. 1 to 4. The electrocardiograph 1 has a horizontally elongate shape and such a size as to be accommodated in the palm. Fig. 1 is a diagram of the electrocardiograph 1 as viewed from a surface thereof having the display functions and the operating functions. By way of explanation, this surface is defined as the front surface and the longitudinal direction of the electrocardiograph 1 is defined as a horizontal direction. Specifically, in Fig. 1, the portion to the right on the front surface is defined as the right side of the electrocardiograph 1, and the portion to the left on the front surface is defined as the left side of the electrocardiograph 1. Fig. 1 is a front view of the electrocardiograph 1, Fig. 2 a top plan view of the electrocardiograph 1, Fig. 3 a left side view of the electrocardiograph 1 and Fig. 4 a right side view of the electrocardiograph 1.

With reference to Fig. 1, the electrocardiograph 1 comprises a display 10 arranged at a position toward the left on the front surface thereof for displaying the measurement results and the guide information. On the right side of the display unit 10, there are arranged a setting switch 11 used for various setting operations, a display switch 12 used for displaying the measurement result on the display unit 10, and a scroll switch 13 for scrolling and displaying the information such as the graph showing the measurement result and the guide information displayed on the display unit 10. Further, on the right side of the front surface, there is arranged a negative electrode 14 over the range corresponding to the vertical side, and a protrusion 15 is formed on the negative electrode 14.

In Figs. 1 to 3, a positive electrode 16 is arranged from the front surface to the back over the left side surface in a range corresponding to the vertical side on the left side of the front surface.

Further, in Fig. 2, at a position rightward on the upper surface and leftward of the negative electrode 14, there are arranged a measurement switch 17 for starting the measurement and a power switch 18 at a position to the right of the measurement switch 17. Also, a neutral electrode 19 making up an indifferent electrode is arranged over a range corresponding to the vertical side in the neighborhood of a position corresponding to the negative electrode 14 on the back. The right end portion of the electrocardiograph 1 including the negative electrode 14 and the neutral electrode 19 makes up a grip for holding the electrocardiograph 1 by the right hand. Also, the left end portion of the electrocardiograph 1 including the positive electrode 16 constitutes a contact section by way of which the electrocardiograph 1 is pressed against the electrode contact portion of the subject.

More specifically, the setting switch 11, the display switch 12 and the scroll switch 13 are each arranged at such a position as to be operated by the thumb of the right hand while the grip including the negative electrode 14 and the neutral electrode 19 is held in the right hand. The measurement switch 17 and the power switch 18, on the other hand, are arranged at such a position as to be operated by the forefinger of the right hand while the grip including the negative electrode 14 and the neutral electrode 19 is held in the right hand. Also, the protrusion 15 is formed to prevent the slipping of the palm when the subject holds the grip including the negative electrode 14 and the neutral electrode 19 by the right hand. The protrusion 14 is thus intended to bring the negative electrode 14 into secure contact with the right hand of the subject.

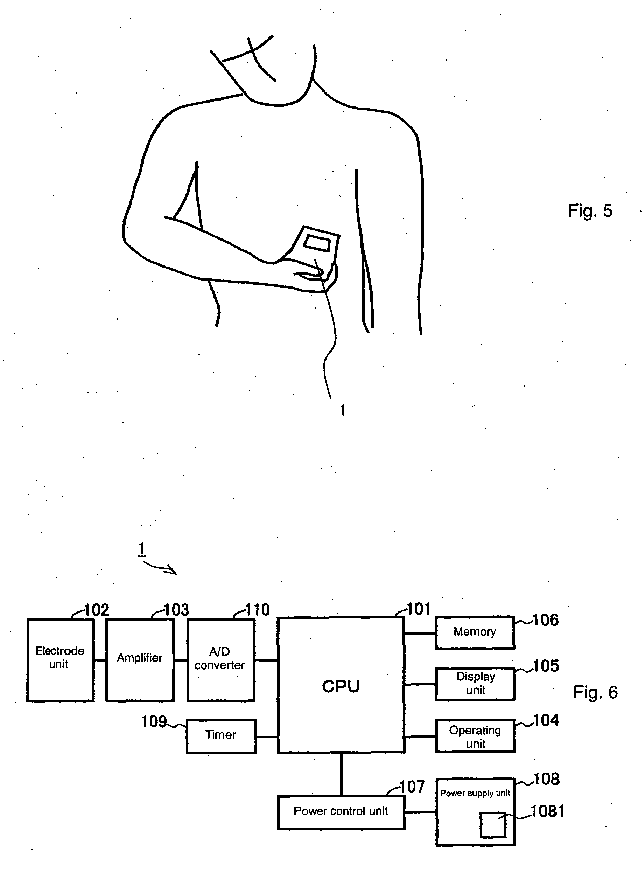

A specific example of the measurement position of the subject for measuring his/her electrocardiographic waveform using the electrocardiograph 1 according to this embodiment is shown in Fig. 5. In Fig. 5, the subject holds the electrocardiograph 1 by holding the grip including the negative electrode 14 and the neutral electrode 19 of the electrocardiograph 1 by the right hand, and measures the electrocardiographic waveform in a position where the contact section including the positive electrode 16 is pressed against the electrode contact portion other than the right hand of the subject. By doing so, a circuit is formed which reaches the right hand in contact with the negative electrode 14 through the heart from the electrode contact portion in contact with the positive electrode 16. Thus, the action potential (electrocardiographic waveform) caused by the electrical excitation generated when the cardiac muscle of the subject is active is measured as a potential difference between the positive electrode 16 and the negative electrode 14 constituting the induction electrodes. The measurement method at the position shown in Fig. 5 represents a typical one, in which the negative electrode 14 is brought into contact with the right hand and the positive electrode 16 is brought into contact with the skin on the fifth intercostal anterior axillary line.

Fig. 6 is a block diagram showing the functional configuration of the electrocardiograph 1 according to this embodiment. In Fig. 6, the electrocardiograph 1 comprises a CPU (central processing unit) 101, an electrode unit 102 including a positive electrode 16 and a negative electrode 14, an amplifier 103, an A/D (analog-to-digital) converter 110, an operating unit 104 including switches 11 to 13, 17, 18, a display unit 105 including a display 10; a memory 106 for storing the measurement data and the program executed by the CPU 101, a power control unit 107, a power supply unit 108 and a timer 109.

The power supply unit 108 supplies power to the electrocardiograph 1. The electrocardiograph 1 assumes two modes including the power save mode in which at least the depression of the switches 11 to 13, 17, 18 can be detected and the power mode in which the program is started and the function corresponding to the depressed switch can be exhibited. The former mode (power save mode) is called the sleep mode, and the latter mode the active mode according to this embodiment. The current mode of the electrocardiograph 1 is stored in the power memory 1081 included in the power supply unit 108.

The CPU 101, upon receipt of a switch signal indicating the depression of the power switch 18 from the operating unit 104 in sleep mode, outputs a control signal instructing the power control unit 107 to supply the starting power. The power control unit 107, in response to the control signal input from the CPU 101, increases the electrical energy supplied by the power supply unit 108 to the starting power level thereby to set the electrocardiograph 1 in active mode.

Once the main power supply of the electrocardiograph 1 is turned on into active mode, the CPU 101 reads and executes the program stored in the memory 106 in accordance with the switch signal input from the operating unit 104, and outputs the control signal to each part of the electrocardiograph 1 thereby to control the overall operation. In the process, the timer 109 may be activated to count as required.

The amplifier 103 detects the potential difference between the positive electrode 16 and the negative electrode 14 and amplifies the voltage to a predetermined amplitude thereby to remove noises. Further, the electrode signal is delivered from the amplifier 103 to the A/D converter 110 and by being converted into a digital signal, input to the CPU 101.

The CPU 101 stores the electrode signal input from the electrode unit 102 through the amplifier 103 and the A/D converter 110 in a predetermined area of the memory 106. Also, the CPU 101 processes the electrode signal input from the amplifier 103 as required for display, generates a measurement result screen including the electrocardiographic waveform and outputs it to the display unit 105. The display unit 105 outputs the measurement result screen input from the CPU 101. Also, the CPU 101 analyzes the input electrode signal and outputs the message information to the display unit 105 to display the message corresponding to the analysis result on the display 10.

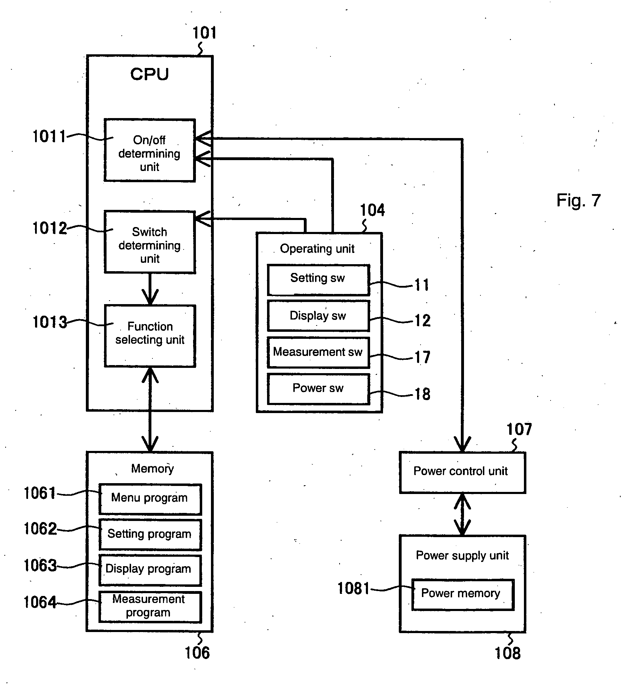

Further, Fig. 7 shows a specific example of the functional configuration of the CPU 101, the operating unit 104, the memory 106, the power control unit 107 and the power supply unit 108. In Fig. 7, as described above, the operating unit 104 includes a setting switch 11 providing a functional switch for designating a function, a display switch 12, a measurement switch 17 and a power switch 18. Upon depression of any one of these switches, a switch signal indicating the particular switch depressed is output to the CPU 101.

The CPU 101 includes an on/off determining unit 1011, a switch determining unit 1012 and a function selecting unit 1013. The on/off determining unit 1011, upon receipt of a switch signal from the operating unit 104, accesses the power memory 1081 of the power supply unit 108 through the power control unit 107 and determines whether the electrocardiograph 1 is in active or sleep mode. In the case where it is determined that the electrocardiograph 1 is in sleep mode, the on/off determining unit 1011 outputs a control signal instructing the power control unit 107 to supply the starting power.

Also, the switch determining unit 1012 analyzes the switch signal input from the operating unit 104 and determines which of the switches is depressed. The switch determining unit 1012 then outputs the determination result to the function selecting unit 1013.

The memory 106 stores the program executed by the CPU 101 in a predetermined area therein as described above. According to this embodiment, the program executed in the CPU 101 may be modularized in accordance with each function of a plurality of processes making up a series of processes executed in the electrocardiograph 1. Specifically, a predetermined area of the memory 106 has stored therein the programs executed by the CPU 101 including a menu program 1061 for causing the CPU 101 to execute the menu process, a setting program 1062 for the CPU 101 to execute the setting process, a display program 1063 for causing the CPU 101 to execute the display process and a measurement program 1064 for causing the CPU 101 to execute the measurement process.

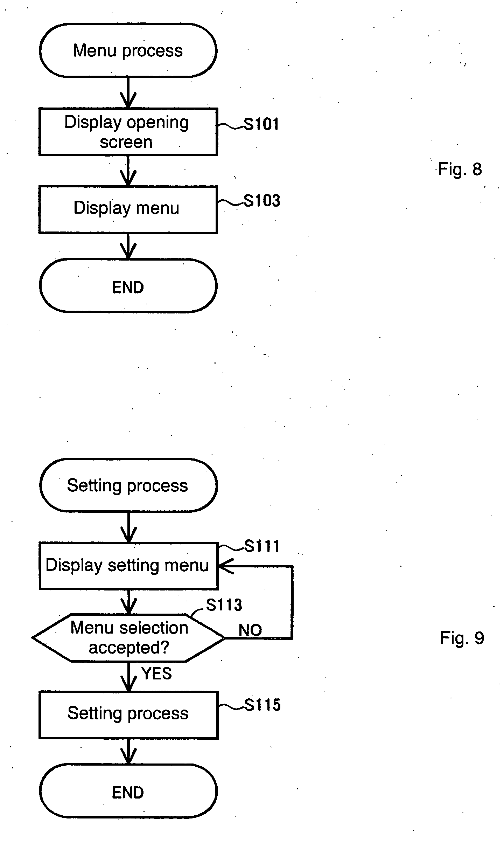

The function selecting unit 1013 of the CPU 101, in accordance with the determination result input from the switch determining unit 1012, i.e. a particular switch depressed, selects a corresponding one of the programs 1061 to 1064 stored in the predetermined area of the memory 106 and determines it as a program to be branched and executed. The process implemented by executing the programs 1061 to 1064 is explained with reference to Figs. 8 to 11.

Referring to Fig. 8, in the menu process implemented by executing the menu program 1061, the CPU 101 first reads the screen information of an opening screen (starting screen) stored in a predetermined area of the memory 106, and outputs it to the display unit 105 to be displayed on the display 10 (S101). Then, the menu screen for selecting subsequent processes is displayed on the display 10 in similar fashion (S103) thereby to accept the selection of the subsequent processes.

Referring to Fig. 9, in the setting process implemented by executing the setting program 1062, the CPU 101 first displays on the display 10 the setting menu to accept the selection of a set item in similar fashion (S111), and accepts the menu selection from the user (YES in S113). The setting process is executed for setting the selected setting item as predetermined (S115). The setting process includes the setting of the display size, the measurement time or the measurement method. These setting methods are widely employed in the computer and therefore are not explained any further.

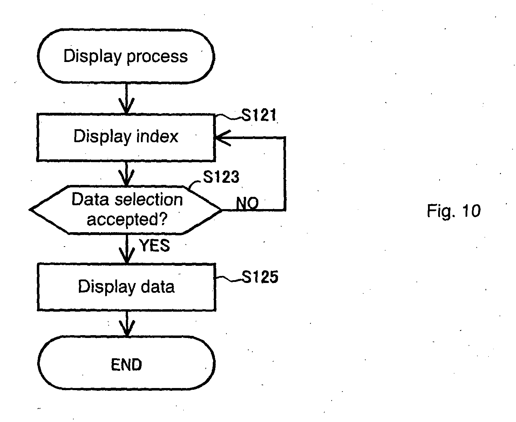

Referring to Fig. 10, in the display process implemented by executing the display program 1063, the CPU 101 first accesses the display data stored in a predetermined area of the memory 106 and displays the index thereof in similar fashion (S121). The display data include the previous measurement result, etc. stored in the memory 106. Once the selection of the data to be displayed is accepted based on the index on display (YES in S123), the corresponding data is read from the memory 106, output to the display unit 105 and displayed on the display 10 (S125).

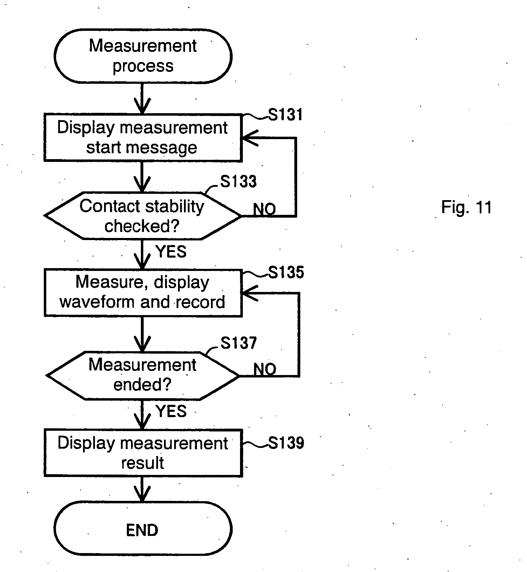

Referring to Fig. 11, in the measurement process implemented by executing the measurement program 1064, the CPU 101 first displays on the display 10 a measurement start message to notify the start of measuring the electrocardiographic wave in similar fashion (S131). Next, based on the electrode signal input from the electrode unit 102, the contact stability indicating whether the positive electrode 16 and/or the negative electrode 14 is in stable contact with a predetermined portion of the subject is measured thereby to assure safety (S133). The process for measuring the contact stability in step S133 is not limited in the invention, but can be the process executed in the conventional electrocardiograph, and therefore will not be explained in detail.

Once the contact stability is confirmed in step S133 (YES in S133), the CPU 101 measures the electrocardiographic waveform of the subject based on the electrode signal input from the electrode unit 102, and displays the measurement result on the display 10 in real time. Also, the measurement result is stored in a predetermined area of the memory 106 (S135). In the case where the contact stability fails to be confirmed in step S133 (NO in S133), on the other hand, the CPU 101 returns the process to step S131. As an alternative, an error notification may be given to the effect that the induction electrode is not in stable contact with a predetermined portion of the subject.

The CPU 101 performs the measurement of step S135 for a predetermined time by referring to the time counted on the timer 109, and ends the measurement (YES in S137) to display the measurement result on the display 10 (S139).

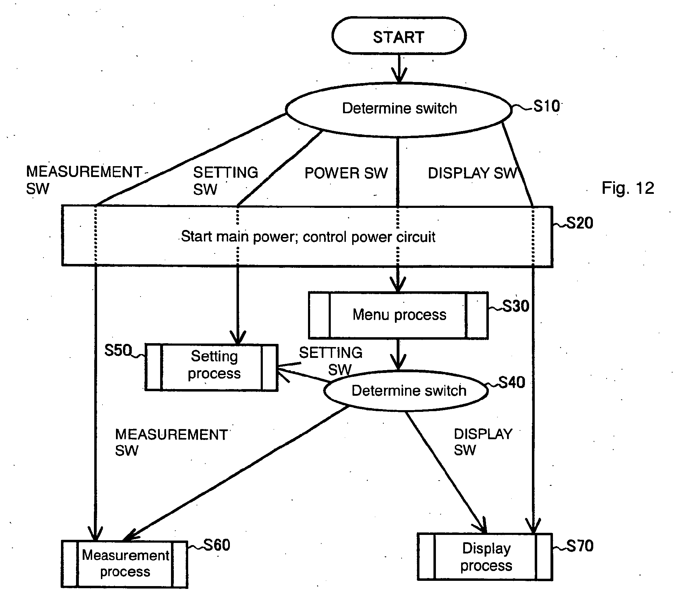

Next, the overall process flow in the electrocardiograph 1 is explained with reference to Fig. 12.

In Fig. 12, assume that one of the switches is depressed in sleep mode. The CPU 101 receives a switch signal indicating the fact from the operating unit 104, and determines the present mode of the electrocardiograph 1 by referring to the power memory 1081 through the on/off determining unit 1011. Also, the switch determining unit 1012 determines which switch has been depressed (S10).

In the case where the electrocardiograph 1 is in sleep mode and the switch determining unit 1012 determines that the power switch 18 is depressed (POWER SW in S10), the main power supply is activated and the electrocardiograph 1 is set in active mode. Then, the CPU 101 executes a predetermined main power starting process, i.e. performs the initial operation of starting to control each part (S20). The function selecting unit 1013 selects the menu program 1061 corresponding to the power switch 18 as a program to be executed, and the process branches to the menu process described above (S30).

In the menu process, the selection by the switch is accepted from the user, and as in step S10, the switch determining unit 1012 determines a particular switch which has been depressed (S40). The function selecting unit 1013 selects, as a program to be executed, the program corresponding to the switch determined to have been depressed by the switch determining unit 1012. Specifically, in the case where the switch determining unit 1012 determines that the setting switch 11 is depressed (SET SW in S40), the function selecting unit 1013 selects the set program 1062 corresponding to the setting switch 11 as a program to be executed, and branches the process to the setting process (S50). In the case where the switch determining unit 1012 determines that the measurement switch 17 is depressed (MEASUREMENT SW in S40), on the other hand, the function selecting unit 1013 selects the measurement program 1064 corresponding to the measurement switch 17 as a program to be executed, and branches the process to the measurement process described above (S60). In the case where the switch determining unit 1012 determines that the display switch 12 is depressed (DISPLAY SW in S40), the function selecting unit 1013 selects the display program 1063 corresponding to the display switch 12 as a program to be executed, and branches the process to the display process described above (S70).

After ending the process in steps S50 to S70 described above, the electrocardiograph 1 may automatically transfer to the sleep mode or the process may be returned to the menu display in step S30.

The ordinary process flow for the electrocardiograph 1 is described above. The electrocardiograph according to this invention has another feature that the next process flow is implemented.

Specifically, in Fig. 12, assume that the switch determining unit 1012 determines that the measurement switch 17 is depressed in sleep mode (MEASUREMENT SW in S10). After the main power supply is turned on and the initial operation is performed in step S20, the measurement program 1064 is selected by the function selecting unit 1013 as a program to be executed, and the process is skipped to step S60 thereby branching to the measurement process described above.

Also assume that the switch determining unit 1012 determines that the setting switch 11 is depressed in sleep mode (SET SW in S10). In similar fashion, the main power supply is turned on and the initial operation performed in step S20, and after that, the function selecting unit 1013 selects the setting program 1062 as a program to be executed, and the process skips to step S50 thereby branching to the setting process described above.

Also in the case where the switch determining unit 1012 determines that the display switch 12 is depressed in sleep mode (DISPLAY SW in S10), the main power supply is turned on and the initial operation is performed similarly in step S20, after which the function selecting unit 1013 selects the display program 1063 as a program to be executed, and the process skips to step S70 thereby branching to the display process described above.

With the electrocardiograph according to this invention, these processes are executed so that the device operation from power on to setting each mode (measurement mode, setting mode, display mode, etc.) is reduced. Also, the time required from power on to setting each mode is shortened. As a result, the time required from power on to measurement is also shortened. In the case where an abnormality such as a chest pain or a palpitation occurs in the subject, the electrocardiographic waveform can be immediately measured and an effective measurement result can be obtained.

### [Modifications]

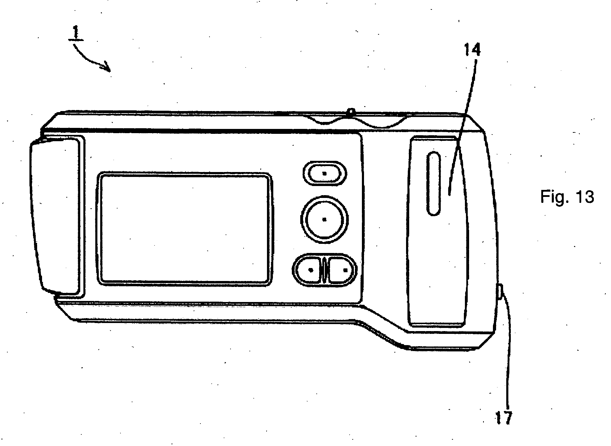

Further, a specific example of the electrocardiograph 1 according to a modification is shown in the front view of Fig. 13. Specifically, according to this modification, the electrocardiograph 1 has the feature that a measurement button 17 is arranged at the grip constituting the right end portion of the electrocardiograph 1 including the electrode 19. The position of the measurement button 17, however, is not limited to the right end of the electrocardiograph 1 as shown in Fig. 13, but may be any other position on the grip or on the contact section.

Also in the electrocardiograph 1 according to the modification, as in the aforementioned case, the main power supply is started by the user operation (by depressing the switch or otherwise) in sleep mode, and the process is skipped and executed from the process corresponding to the user operation.

With this configuration of the electrocardiograph 1 according to the modification, the subject holds the grip of the electrocardiograph 1 by the right hand when starting the measurement of the electrocardiographic wave or presses the contact section against the electrode contact portion. Then, the measurement button 17 is depressed and the process is skipped from power on to the measurement mode. Specifically, this configuration of the electrocardiograph according to the modification and the process executed as described above reduces the device operation steps from power on to measurement. Thus, the time required from power on to measurement is shortened. As a result, in the case where an abnormality such as the chest pain or palpitation of the subject occurs, the electrocardiographic waveform can be measured immediately and an effective measurement result can be obtained.

In the method of controlling the electrocardiograph according to the invention, the main power supply is switched on by the user operation (depression of the switch, etc.) in sleep mode and the process before the one corresponding to the user operation is skipped so that the execution is started from the process corresponding to the user operation. This method is applicable also to other apparatuses as well as the electrocardiograph. Other apparatuses specifically include a PDA (personal digital assistance), a mobile phone, an electronic dictionary, and a recording medium reproducing device such as a DVD (digital video disk) player.

The embodiments disclosed above should be considered illustrative and not limitative in all respects. The scope of this invention is not limited to the foregoing description but defined by the appended claims and is intended to cover all modifications thereof without departing from the scope and spirit of the invention.

## Claims

1. An electrocardiograph comprising:
a functional switch for designating a function;
a storage device for storing a program to implement the function; and
a power control device for controlling the power supply; and
a control device wherein upon detection of the depression of the functional switch in power save mode where at least the depression of the functional switch can be detected, the control device controls the power control device to supply power to achieve the power mode capable of implementing the function, and performs control operation to execute the program stored in the storage device for implementing the function corresponding to the functional switch of which the depression has been detected.

2. An electrocardiograph according to claim 1, wherein the function includes the function of measuring the electrocardiographic wave in the electrocardiograph.

3. An electrocardiograph according to claim 2, further comprising at least a pair of induction electrodes for measuring the electrocardiographic waveform of the subject by contacting the body of the subject,
wherein the measurement switch making up the functional switch for designating the function of measuring the electrocardiographic waveform is arranged at a position adapted to be depressed when the induction electrodes are brought into contact with the body of the subject, and
wherein once the induction electrodes are brought into contact with the body of the subject when power is off, the control device controls the power control device to supply power and performs control operation to execute the program stored in the storage device to implement the measurement function.

4. An electrocardiograph control method for controlling an electrocardiograph including a functional switch for designating a function and a storage device for storing a program to implement the function, comprising:
the power control step for controlling the power supply;
the first control step for executing the power control step to supply power until the power mode capable of implementing the function is attained, upon detection of depression of the functional switch in power save mode where at least the depression of the functional switch can be detected; and
the second control step for performing control operation to execute the program stored in the storage device to implement the function corresponding to the functional switch of which the depression has been detected.
